# EUROPEAN PATENT APPLICATION

(11) **EP 4 212 144 A1**
(43) Date of publication of application: **19.07.2023**
(21) Application number: 21864639.6
(22) Date of filing: 01.09.2021
(51) Int. Cl.: A61K 8/06, A61K 8/34, A61K 8/55, A61K 8/60, A61K 8/92, A61Q 17/00, A01N 25/04, A01N 31/02

(54) **SANITIZER COMPOSITION IN EMULSION FORMULATION**

(30) Priority: 07.09.2020 KR 20200113608; 26.08.2021 KR 20210113325
(71) Applicant: Amorepacific Corporation, Seoul 04386 (KR)
(72) Inventor: SEO, Bohyun, Yongin-si, Gyeonggi-do 17074 (KR); KIM, Daekyeong, Yongin-si, Gyeonggi-do 17074 (KR); KIM, Chanho, Yongin-si, Gyeonggi-do 17074 (KR); JUNG, Chang Jo, Yongin-si, Gyeonggi-do 17074 (KR); CHAE, Sangkyun, Yongin-si, Gyeonggi-do 17074 (KR)
(74) Representative: Dr. Schön, Neymeyr & Partner Patentanwälte mbB
(86) International application number: PCT/KR2021/011750
(87) International publication number: WO 2022/050680

(57) **Abstract**

The present application discloses a sanitizer composition in an emulsion formulation. The sanitizer composition comprises: ethanol; a surfactant; oil; and water. The ethanol content is greater than 10% by weight on the basis of the total weight of the sanitizer composition. The surfactant may be one or more of a lecithin-based surfactant and a sorbitan-based surfactant, and the oil may be one or more selected from the group consisting of C12-15 alkyl benzoate, coconut oil, olive oil, and palm oil. The sanitizer composition has an effect of providing a high moisturizing feeling while comprising a large amount of ethanol, and of implementing an emulsion formulation such as a lotion.

## Description

### [Technical Field]

### [Cross-Reference to Related Applications]

The present application claims priority to Korean Patent Application No. 10-2020-0113608 filed on September 7, 2020 and Korean Patent Application No. 10-2021-0113325 filed on August 26, 2021 in the Republic of Korea, the disclosures of which are incorporated herein by reference.

Disclosed is an emulsion-type disinfectant composition.

### [Background Art]

The most important thing in individual hygiene is cleanliness of hands exposed frequently to bacteria and viruses. Therefore, it is important to wash hands frequently with flowing water or to prevent bacterial and viral infection by using a hand disinfectant in a water-free environment. A hand disinfectant is a kind of disinfectant provided to disinfect hands exposed frequently to bacteria and viruses in a simple manner. In general, hand disinfectants using ethanol have been used most widely.

However, there is a problem in that O/W emulsion formulations cannot be formed with ease, when oil is added to prepare hand disinfectants using ethanol. Therefore, conventional hand disinfectants using ethanol have a limitation in that they are provided in liquid or gel formulations. Particularly, purified water or a moisturizer ingredient, such as glycerin, is added to ethanol to form a liquid formulation, or a thickener ingredient is added to ethanol to form a gel formulation. In addition to such formulations, there is a product made in the form of spray.

In addition, hand disinfectants using ethanol cause skin irritation or skin dryness due to ethanol. In a severe case, one's skin stings and skin shell peeling occur, and thus use of such hand disinfectants has a low preference. Particularly, some people reject use of such hand disinfectants, because the disinfectants have a bad feel. Therefore, there is a need for developing a novel hand disinfectant product required to be used frequently in daily life in order to protect individual hygiene and to prevent bacterial and viral infection, and particularly having a significantly improved preference of consumers or users.

### [Disclosure]

### [Technical Problem]

In one aspect, the present disclosure is directed to providing an emulsion-type disinfectant composition.

In another aspect, the present disclosure is directed to providing a disinfectant composition including ethanol and oil and having an emulsion formulation.

In still another aspect, the present disclosure is directed to providing a disinfectant composition including ethanol and oil and having an O/W emulsion formulation.

In still another aspect, the present disclosure is directed to providing an emulsion-type disinfectant composition including 10 wt% or more of ethanol.

In still another aspect, the present disclosure is directed to providing an emulsion-type disinfectant composition including ethanol at a high content, such as 30 wt% or more, 40 wt% or more, 50 wt% or more, or 60 wt%.

In still another aspect, the present disclosure is directed to providing a disinfectant composition including ethanol at a high content, such as 30 wt% or more, 40 wt% or more, 50 wt% or more, or 60 wt%, and having an O/W emulsion formulation.

In still another aspect, the present disclosure is directed to providing an emulsion-type disinfectant composition including ethanol and having an excellent skin moisturizing ability.

### [Technical Solution]

In one aspect, there is provided an emulsion-type disinfectant composition including: ethanol; a surfactant; oil; and water, wherein the content of ethanol is more than 10 wt% based on the total weight of the disinfectant composition.

### [Advantageous Effects]

In one aspect, the present disclosure provides an emulsion-type disinfectant composition.

### [Brief Description of Drawings]

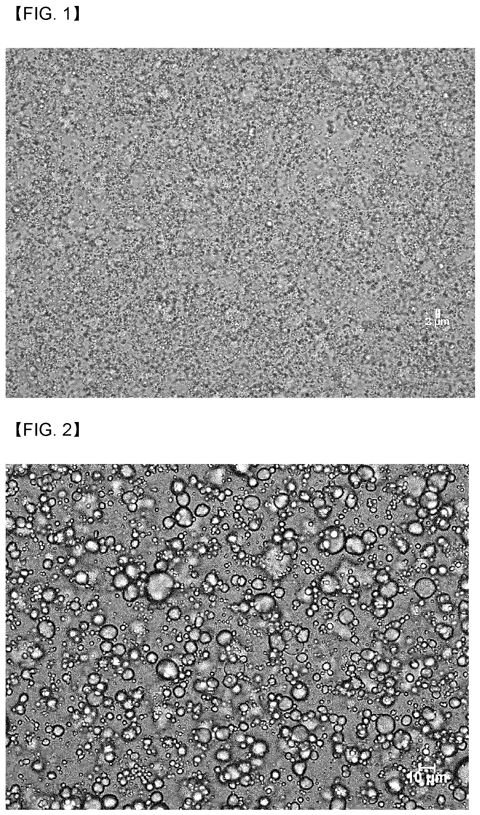

FIG. 1 shows a photographic image of the emulsion particles of the disinfectant composition according to Example 2-1 of the present disclosure, as observed by an optical microscope. It can be seen that the disinfectant composition according to Example 2-1 forms uniform and stable emulsion particles having an average size of 1-5 µm.
FIG. 2 shows a photographic image of the emulsion particles of the disinfectant composition according to Comparative Example 2-5, as observed by an optical microscope. It can be seen that the disinfectant composition according to Comparative Example 2-5 causes an oil separation phenomenon and forms unstable and non-uniform emulsion particles having an average size of more than 10 µm.

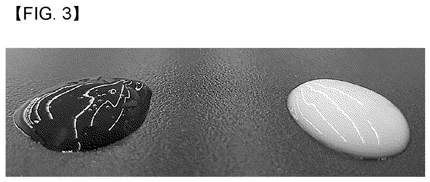

FIG. 3 shows a photographic image illustrating the result of comparison of a commercially available transparent gel-type hand disinfectant composition (the left-side image) with the disinfectant composition (the right-side image) according to Examples 2-1 of the present disclosure, as observed by the naked eyes. It can be seen that the commercially available hand disinfectant composition looks black, because it has a transparent gel formulation and directly reflects the black color of the background surface, while the disinfectant composition according to Example 2-1 has a milky-colored lotion-type O/W emulsion formulation.

### [Best Mode]

Exemplary embodiments now will be described more fully hereinafter.

In one aspect of the present disclosure, there is provided an emulsion-type disinfectant composition including: ethanol; a surfactant; oil; and water, wherein the content of ethanol is more than 10 wt% based on the total weight of the disinfectant composition.

In another aspect of the present disclosure, there is provided use of a combination in manufacturing an emulsion-type disinfectant composition, wherein the combination includes: ethanol; a surfactant; oil; and water, and the content of ethanol is more than 10 wt% based on the total weight of the disinfectant composition.

In still another aspect of the present disclosure, there is provided use of a composition for disinfection and/or moisturization, wherein the composition has an emulsion formulation and includes: ethanol; a surfactant; oil; and water, and the content of ethanol is more than 10 wt% based on the total weight of the disinfectant composition.

In still another aspect of the present disclosure, there is provided a disinfection and/or moisturization method, which includes applying a composition in an amount effective for disinfection and/or moisturization to a subject in need thereof, wherein the composition has an emulsion composition and includes: ethanol; a surfactant; oil; and water, and the content of ethanol is more than 10 wt% based on the total weight of the composition.

In yet another aspect of the present disclosure, there is provided a method for preparing an emulsion-type disinfectant composition, including a step of mixing: ethanol; a surfactant; oil; and water, wherein the content of ethanol is more than 10 wt% based on the total weight of the disinfectant composition.

According to an embodiment, the disinfection may be skin disinfection.

According to an embodiment, the disinfection may be hand disinfection.

According to an embodiment, the content of ethanol may be 20 wt% or more, 30 wt% or more, 55 wt% or more, 60 wt% or more, 65 wt% or more, or 70 wt% or more, based on the total weight of the disinfectant composition.

According to another embodiment, the content of ethanol may be more than 10 wt%, 15 wt% or more, 20 wt% or more, 25 wt% or more, 30 wt% or more, 35 wt% or more, 40 wt% or more, 45 wt% or more, 50 wt% or more, 55 wt% or more, 60 wt% or more, 65 wt% or more, 70 wt% or more, or 75 wt% or more, and less than 80 wt%, 75 wt% or less, 70 wt% or less, 65 wt% or less, 60 wt% or less, 55 wt% or less, 50 wt% or less, 45 wt% or less, 40 wt% or less, 35 wt% or less, 30 wt% or less, or 25 wt% or less, based on the total weight of the disinfectant composition.

According to still another embodiment, the content of ethanol may be more than 10 wt% and less than 80 wt%, based on the total weight of the disinfectant composition.

According to still another embodiment, the content of ethanol may be 20 wt% or more and less than 80 wt%, based on the total weight of the disinfectant composition.

According to still another embodiment, the content of ethanol may be 20-75 wt%, based on the total weight of the disinfectant composition.

According to still another embodiment, the content of ethanol may be 30-75 wt%, based on the total weight of the disinfectant composition.

According to still another embodiment, the content of ethanol may be 40-75 wt%, based on the total weight of the disinfectant composition.

According to yet another embodiment, the content of ethanol may be 50-75 wt%, based on the total weight of the disinfectant composition.

According to an embodiment, the surfactant may be at least one of a lecithin-based surfactant and a sorbitan-based surfactant.

According to an embodiment, the lecithin-based surfactant may be at least one selected from the group consisting of lecithin, hydrogenated lecithin and synthetic lecithin.

According to an embodiment, the lecithin-based surfactant may be lecithin.

According to an embodiment, the sorbitan-based surfactant may be a sorbitan fatty acid ester.

According to an embodiment, the sorbitan-based surfactant may be at least one selected from the group consisting of sorbitan olivate, sorbitan stearate, sorbitan isostearate, sorbitan tristearate, sorbitan sesquistearate, sorbitan palmitate, sorbitan laurate, sorbitan oleate, sorbitan sesquioleate, sorbitan trioleate, sorbitan stearate/sorbityl laurate, polyoxyethylene sorbitan monolaurate, Polysorbate 20, Polysorbate 40, Polysorbate 60, Polysorbate 61, Polysorbate 65, Polysorbate 80 and Polysorbate 85.

According to an embodiment, the sorbitan-based surfactant may be polyoxyethylene sorbitan monolaurate.

According to an embodiment, the surfactant may be at least one of lecithin and polyoxyethylene sorbitan monolaurate.

According to an embodiment, the surfactant may be used in an amount of more than 0.1 wt% and less than 5 wt%, based on the total weight of the disinfectant composition.

According to another embodiment, the surfactant may be used in an amount of more than 0.1 wt%, 0.13 wt% or more, 0.15 wt% or more, 0.17 wt% or more, 0.2 wt% or more, 0.23 wt% or more, 0.25 wt% or more, 0.27 wt% or more, 0.3 wt% or more, 0.33 wt% or more, 0.35 wt% or more, 0.37 wt% or more, 0.4 wt% or more, 0.43 wt% or more, 0.45 wt% or more, 0.47 wt% or more, 0.5 wt% or more, 0.53 wt% or more, 0.55 wt% or more, 0.57 wt% or more, 0.6 wt% or more, 0.63 wt% or more, 0.65 wt% or more, 0.67 wt% or more, 0.7 wt% or more, 0.73 wt% or more, 0.75 wt% or more, 0.77 wt% or more, 0.8 wt% or more, 0.83 wt% or more, 0.85 wt% or more, 0.87 wt% or more, 0.9 wt% or more, 0.93 wt% or more, 0.95 wt% or more, 0.97 wt% or more, 1.0 wt% or more, 1.3 wt% or more, 1.5 wt% or more, 1.7 wt% or more, or 2 wt% or more, and less than 5 wt%, 4.7 wt% or less, 4.5 wt% or less, 4.3 wt% or less, 4 wt% or less, 3.7 wt% or less, 3.5 wt% or less, 3.3 wt% or less, 3 wt% or less, 2.7 wt% or less, 2.5 wt% or less, 2.3 wt% or less, 2 wt% or less, 1.7 wt% or less, 1.5 wt% or less, 1.3 wt% or less, or 1 wt% or less, based on the total weight of the disinfectant composition.

According to still another embodiment, the surfactant may be used in an amount of 0.2-4 wt%, based on the total weight of the disinfectant composition.

According to still another embodiment, the surfactant may be used in an amount of 0.3-3 wt%, based on the total weight of the disinfectant composition.

According to still another embodiment, the surfactant may be used in an amount of 0.5-2 wt%, based on the total weight of the disinfectant composition.

According to yet another embodiment, the surfactant may be used in an amount of 1-2 wt%, based on the total weight of the disinfectant composition.

According to an embodiment, the oil may be at least one selected from the group consisting of C12-C15 alkyl benzoates, coconut oil, olive oil and palm oil.

According to an embodiment, the oil may be a C12-C15 alkyl benzoate.

According to an embodiment, the oil may be used in an amount of more than 1 wt% and less than 16 wt%, based on the total weight of the disinfectant composition.

According to another embodiment, the oil may be used in an amount of more than 1 wt%, 1.2 wt% or more, 1.4 wt% or more, 1.5 wt% or more, 1.6 wt% or more, 1.8 wt% or more, 2 wt% or more, 2.2 wt% or more, 2.4 wt% or more, 2.5 wt% or more, 2.6 wt% or more, 2.8 wt% or more, 3 wt% or more, 3.2 wt% or more, 3.4 wt% or more, 3.5 wt% or more, 3.6 wt% or more, 3.8 wt% or more, 4 wt% or more, 4.2 wt% or more, 4.4 wt% or more, 4.5 wt% or more, 4.6 wt% or more, 4.8 wt% or more, 5 wt% or more, 5.2 wt% or more, 5.4 wt% or more, 5.5 wt% or more, 5.6 wt% or more, 5.8 wt% or more, or 6 wt% or more, and less than 16 wt%, 15.8 wt% or less, 15.6 wt% or less, 15.4 wt% or less, 15.2 wt% or less, 15 wt% or less, 14.8 wt% or less, 14.6 wt% or less, 14.4 wt% or less, 14.2 wt% or less, 14 wt% or less, 13.8 wt% or less, 13.6 wt% or less, 13.4 wt% or less, 13.2 wt% or less, 13 wt% or less, 12.8 wt% or less, 12.6 wt% or less, 12.4 wt% or less, 12.2 wt% or less, 12 wt% or less, 11.8 wt% or less, 11.6 wt% or less, 11.4 wt% or less, 11.2 wt% or less, 11 wt% or less, 10.8 wt% or less, 10.6 wt% or less, 10.4 wt% or less, 10.2 wt% or less, 10 wt% or less, 9.8 wt% or less, 9.6 wt% or less, 9.4 wt% or less, 9.2 wt% or less, 9 wt% or less, 8.8 wt% or less, 8.6 wt% or less, 8.4 wt% or less, 8.2 wt% or less, 8 wt% or less, 7.8 wt% or less, 7.6 wt% or less, 7.4 wt% or less, 7.2 wt% or less, 7 wt% or less, 6.8 wt% or less, 6.6 wt% or less, 6.4 wt% or less, 6.2 wt% or less, 6 wt% or less, 5.8 wt% or less, 5.6 wt% or less, 5.4 wt% or less, 5.2 wt% or less, or 5 wt% or less, based on the total weight of the disinfectant composition.

According to another embodiment, the oil may be used in an amount of 2-15 wt%, based on the total weight of the disinfectant composition.

According to still another embodiment, the oil may be used in an amount of 2-6 wt%, based on the total weight of the disinfectant composition.

According to an embodiment, the surfactant and the oil are present in a ratio of 0.3 to 3 : 5 based on the weight ratio of the surfactant and oil components.

According to an embodiment, the disinfectant composition may further include at least one additive selected from a thickener, polyol and a pH modifier.

According to an embodiment, the combination may further include at least one additive selected from a thickener, polyol and a pH modifier.

According to an embodiment, the thickener may be at least one selected from the group consisting of guar gum, xanthan gum, gellan gum, carrageenan gum, cellulose, agar, pectin, Carbomer, sodium magnesium silicate, hydroxyethyl cellulose, hydroxypropyl starch phosphate and hydroxyethyl acrylate/sodium acryloyldimethyl taurate copolymers.

According to an embodiment, the polyol may be at least one selected from the group consisting of glycerin, butylene glycol, dipropylene glycol, pentylene glycol, hexylene glycol, diethylene glycol, propanediol, caprylyl glycol, methyl propanediol, Polybutylene glycol-10, Polyglycerin-3, Polyglycerin-4, Polyglycerin-6, Polyglycerin-10, Polyglycerin-20, Polyglycerin-40, Sorbeth-5, Sorbeth-6, Sorbeth-20, Sorbeth-30, Sorbeth-40, dihydroxypropyl PG-glucoside, dithiaoctanediol, fructose, glucamin, methyl glucamin, glucose, Methylgluceth-10, Methylgluceth-20, phytantriol, thioglycerin, diglycerin, sorbitol, erythritol, pentaerythritol, inositol, mannitol, maltose, mannan, maltitol, mannose, lactitol, lactose, threitol, trimethylol propane, and xylitol.

According to an embodiment, the pH modifier may be at least one selected from the group consisting of phosphoric acid and salts thereof, lactic acid and salts thereof, glycolic acid and salts thereof, citric acid and salts thereof, acetic acid, ammonia, monoethanolamine, triethanolamine, potassium hydroxide, sodium hydroxide, sodium carbonate and aminomethyl propanol.

According to an embodiment, the additive may be used in an amount of 0.5-2.0 wt%, based on the total weight of the disinfectant composition.

According to an embodiment, the disinfectant composition may have a viscosity of 100-10,000 cps at 30°C. The disinfectant composition disclosed herein may form an O/W emulsion formulation having a viscosity of more than about 6,000 cps. This suggests that the disinfectant composition disclosed herein may have a higher viscosity as compared to the conventional liquid-type disinfectant compositions having a viscosity of less than about 100 cps, or the conventional gel-type disinfectant compositions having a viscosity of about 2,000-6,000 cps. When the conventional disinfectant compositions include ethanol, particularly in a large amount, it is difficult to form stabilized O/W emulsion formulations by adding oil thereto. In one aspect, the present disclosure provides the effects of providing a disinfectant composition using ethanol in an O/W emulsion formulation by adding oil and realizing an excellent moisturizing ability.

According to another embodiment, the disinfectant composition may have a viscosity of 100 cps or more, 200 cps or more, 300 cps or more, 400 cps or more, 500 cps or more, 600 cps or more, 700 cps or more, 800 cps or more, 900 cps or more, 1,000 cps or more, 2,000 cps or more, 3,000 cps or more, 4,000 cps or more, 5,000 cps or more, 6,000 cps or more, or more than 6,000 cps, and 10,000 cps or less, 9,000 cps or less, 8,000 cps or less, 7,000 cps or less, 6,000 cps or less, 5,000 cps or less, 4,000 cps or less, 3,000 cps or less, 2,000 cps or less, or 1,000 cps or less at 30°C.

According to another embodiment, the disinfectant composition may have a viscosity of 500-9,000 cps, 1,000-9,000 cps, 2,000-9,000 cps, 500-5,000 cps, 1,000-3,000 cps, or 2,000-3,000 cps at 30°C.

According to an embodiment, the disinfectant composition may have emulsion particles having an average size of 1-10 µm. Herein, 'average size' may refer to 'average particle diameter'.

According to another embodiment, the disinfectant composition may have emulsion particles having an average size of 1 µm or more, 2 µm or more, 3 µm or more, 4 µm or more, or 5 µm or more, and 10 µm or less, 9 µm or less, 8 µm or less, 7 µm or less, 6 µm or less, 5 µm or less, 4 µm or less, 3 µm or less, or 2 µm or less.

According to still another embodiment, the disinfectant composition may have emulsion particles having an average size of 1-5 µm, 2-5 µm, or 1-3 µm.

According to an embodiment, the composition may be a composition for disinfection and/or moisturization.

According to an embodiment, the composition may be composition for disinfection and/or moisturization having an O/W emulsion formulation.

According to an embodiment, the disinfectant composition may be an O/W emulsion formulation.

According to an embodiment, the disinfectant composition may be essence or lotion having an O/W emulsion formulation.

According to an embodiment, the disinfectant composition may be a skin disinfectant composition.

According to an embodiment, the disinfectant composition may be a hand disinfectant composition.

According to an embodiment, the disinfectant composition may be a composition for external use on skin.

According to an embodiment, the disinfectant composition may be a cosmetic composition.

According to an embodiment, the disinfectant composition may be a sanitary-aid composition.

The following embodiments are provided by the present disclosure.

Embodiment 1. An emulsion-type disinfectant composition including: ethanol; a surfactant; oil; and water, wherein the content of ethanol is more than 10 wt% based on the total weight of the disinfectant composition.

Embodiment 2. The emulsion-type disinfectant composition as defined in Embodiment 1, wherein the content of ethanol is 20 wt% or more, 30 wt% or more, 55 wt% or more, 60 wt% or more, 65 wt% or more, or 70 wt% or more, based on the total weight of the disinfectant composition.

Embodiment 3. The emulsion-type disinfectant composition as defined in Embodiment 1 or 2, wherein the surfactant is at least one of a lecithin-based surfactant and a sorbitan-based surfactant.

Embodiment 4. The emulsion-type disinfectant composition as defined in Embodiment 3, wherein the lecithin-based surfactant is at least one selected from the group consisting of lecithin, hydrogenated lecithin and synthetic lecithin.

Embodiment 5. The emulsion-type disinfectant composition as defined in Embodiment 3, wherein the sorbitan-based surfactant is a sorbitan fatty acid ester.

Embodiment 6. The emulsion-type disinfectant composition as defined in Embodiment 3, wherein the sorbitan-based surfactant is at least one selected from the group consisting of sorbitan olivate, sorbitan stearate, sorbitan isostearate, sorbitan tristearate, sorbitan sesquistearate, sorbitan palmitate, sorbitan laurate, sorbitan oleate, sorbitan sesquioleate, sorbitan trioleate, sorbitan stearate/sorbityl laurate, polyoxyethylene sorbitan monolaurate, Polysorbate 20, Polysorbate 40, Polysorbate 60, Polysorbate 61, Polysorbate 65, Polysorbate 80 and Polysorbate 85.

Embodiment 7. The emulsion-type disinfectant composition as defined in any one of Embodiment 1 to Embodiment 6, wherein the surfactant is used in an amount of more than 0.1 wt% and less than 5 wt%, based on the total weight of the disinfectant composition.

Embodiment 8. The emulsion-type disinfectant composition as defined in any one of Embodiment 1 to Embodiment 7, wherein the oil is at least one selected from the group consisting of C12-C15 alkyl benzoates, coconut oil, olive oil and palm oil.

Embodiment 9. The emulsion-type disinfectant composition as defined in any one of Embodiment 1 to Embodiment 8, wherein the oil is used in an amount of more than 1 wt% and less than 16 wt%, based on the total weight of the disinfectant composition.

Embodiment 10. The emulsion-type disinfectant composition as defined in any one of Embodiment 1 to Embodiment 9, wherein the surfactant and the oil are present in a ratio of 0.3 to 3 : 5 based on the weight ratio of the surfactant and oil components.

Embodiment 11. The emulsion-type disinfectant composition as defined in any one of Embodiment 1 to Embodiment 10, which further includes at least one additive selected from a thickener, polyol and a pH modifier.

Embodiment 12. The emulsion-type disinfectant composition as defined in Embodiment 11, wherein the additive is used in an amount of 0.5-2.0 wt%, based on the total weight of the disinfectant composition.

Embodiment 13. The emulsion-type disinfectant composition as defined in any one of Embodiment 1 to Embodiment 12, which has a viscosity of 100-10,000 cps.

Embodiment 14. The emulsion-type disinfectant composition as defined in any one of Embodiment 1 to Embodiment 13, which has emulsion particles having an average size of 1-10 µm.

Embodiment 15. The emulsion-type disinfectant composition as defined in any one of Embodiment 1 to Embodiment 14, which is an O/W emulsion formulation.

Embodiment 16. The emulsion-type disinfectant composition as defined in any one of Embodiment 1 to Embodiment 15, which is a skin disinfectant composition.

Embodiment 17. The emulsion-type disinfectant composition as defined in any one of Embodiment 1 to Embodiment 16, which is a hand disinfectant composition.

Embodiment 18. The emulsion-type disinfectant composition as defined in any one of Embodiment 1 to Embodiment 17, which is a composition for external use on skin.

Embodiment 19. An emulsion-type disinfectant composition including: ethanol; at least one surfactant selected from a lecithin-based surfactant and a sorbitan-based surfactant; at least one oil selected from C12-C15 alkyl benzoate, coconut oil, olive oil and palm oil; and water, wherein the content of ethanol is more than 10 wt% and less than 80 wt%, based on the total weight of the disinfectant composition.

Embodiment 20. An emulsion-type disinfectant composition including: ethanol; at least one surfactant selected from a lecithin-based surfactant and a sorbitan-based surfactant; at least one oil selected from C12-C15 alkyl benzoate, coconut oil, olive oil and palm oil; and water, wherein the content of ethanol is 20-75 wt%, based on the total weight of the disinfectant composition.

Embodiment 21. An emulsion-type disinfectant composition including: ethanol; at least one surfactant selected from a lecithin-based surfactant and a sorbitan-based surfactant; at least one oil selected from C12-C15 alkyl benzoate, coconut oil, olive oil and palm oil; and water, wherein the content of ethanol is 20-75 wt% based on the total weight of the disinfectant composition, the content of the surfactant is 0.3-3 wt% based on the total weight of the disinfectant composition, and the content of the oil is 2-15 wt% based on the total weight of the disinfectant composition.

Embodiment 22. An emulsion-type disinfectant composition including: ethanol; at least one surfactant selected from lecithin and a sorbitan fatty acid ester; at least one oil selected from C12-C15 alkyl benzoate, coconut oil, olive oil and palm oil; and water, wherein the content of ethanol is 20-75 wt% based on the total weight of the disinfectant composition, the content of the surfactant is 0.3-3 wt% based on the total weight of the disinfectant composition, and the content of the oil is 2-15 wt% based on the total weight of the disinfectant composition.

Embodiment 23. An emulsion-type disinfectant composition including: ethanol; at least one surfactant selected from lecithin and polyoxyethylene sorbitan monolaurate; at least one oil selected from C12-C15 alkyl benzoate, coconut oil, olive oil and palm oil; and water, wherein the content of ethanol is 20-75 wt% based on the total weight of the disinfectant composition, the content of the surfactant is 0.3-3 wt% based on the total weight of the disinfectant composition, and the content of the oil is 2-15 wt% based on the total weight of the disinfectant composition.

Embodiment 24. An emulsion-type disinfectant composition including, based on the total weight of the disinfectant composition, 15 to 75 wt% of ethanol; more than 0.1 to less than 5.0 wt% of a surfactant selected from the group consisting of a lecithin-based surfactant and a sorbitan-based surfactant; more than 1.0 to less than 16.0 wt% of an oil selected from the group consisting of a C12-C15 alkyl benzoate, coconut oil, olive oil and palm oil; and the balance water, wherein the surfactant and the oil are present in a ratio of 0.3 to 3 : 5 based on the weight ratio of the surfactant and oil components.

### [Modes for Invention]

Exemplary embodiments now will be described more fully hereinafter. The present disclosure may, however, be embodied in many different forms, and it will be apparent to those skilled in the art that the scope of the present disclosure should not be construed as limited to the exemplary embodiments set forth therein.

### Example 1. Evaluation of Formulation Stability and Disinfection Power Depending on Change in Ethanol Content

Each disinfectant composition having an O/W emulsion formulation was prepared according to the composition (wt%) of the following Table 1 in the conventional manner.
1) The aqueous phase part ingredients were warmed to 50-75°C, mixed with one another and dissolved.
2) The remaining oil phase part ingredients were warmed to 50-75°C, mixed with one another and dissolved.
3) The aqueous phase part was mixed with the oil phase part, while maintaining the temperature at 50-75°C, and a disinfectant composition having an O/W emulsion formulation was prepared by using a homogenizer.

In addition, each composition prepared as described above was evaluated in terms of formulation stability and disinfection power as follows.

The formulation stability was evaluated by storing each composition 1) at 4°C for 2 weeks, 2) at 40°C for 2 weeks, 3) at 60°C for 2 weeks, and then 4) under a cycle condition of increasing and reducing temperature within a range of temperature of 4-60°C at a predetermined rate for 2 weeks. If oil separation occurs in a composition under any one of the test conditions, the formulation stability of the composition is marked as 'unstable'. If no oil separation occurs in a composition under all of the test conditions, the formulation stability of the composition is marked as 'stable'. In addition, stabilized emulsion particles were compared with unstable and non-uniform emulsion particles causing oil separation through an optical microscope (ECLIPSE NI-U, Nikon Corporation) (see, FIG. 1 and FIG. 2).

As a result, it can be seen that when the ethanol content in a composition reaches 80 wt%, the composition shows low formulation stability. Therefore, it can be seen that the disinfectant composition having an O/W emulsion formulation of the present disclosure according to one aspect shows excellent formulation stability, when it includes ethanol in an amount of less than 80 wt%, particularly 75 wt% or less, based on the total weight of the composition.

The disinfection power was evaluated by inoculating TSB (Tryptic soy broth) medium with *Staphylococcus aureus* (ATCC 6538), culturing *Staphylococcus aureus* at 35°C for 24 hours, and by using a dilution of the culture solution with PBS as a test bacterial solution. Each composition as a test sample was dissolved in PBS, introduced to a 96-well plate, inoculated with 1×10⁶ CFU/mL of the prepared test bacterial solution, and then the mortality rate of *Staphylococcus aureus* 1 minute after the inoculation was determined and marked.

As a result, it can be seen that when a composition has an ethanol content of more than 10 wt%, particularly 20 wt% or more, based on the total weight of the composition, it shows a mortality rate of 99.999% within 1 minute. Therefore, it can be seen that when a composition has an ethanol content of more than 10 wt%, particularly 20 wt% or more, based on the total weight of the composition, it can be used as a disinfectant.

Further, the viscosity of each composition was determined by using a viscometer (DV2TLVTJ0, Brookfield Viscometer, USA) equipped with NO. 73 spindle under the condition of 12 rpm at 30°C for 2 minutes. As a result, it can be seen that the composition having a relatively low ethanol content according to Comparative Example 1-1 has a viscosity of about 10,000 cps, and the composition having a relatively high ethanol content according to Comparative Example 1-2 has a viscosity of about 100 cps. It is shown that each of the compositions according to Examples 1-1 to 1-6 has a viscosity of about 1,000-8,700 cps depending on a change in ethanol content.

**[Table 1]**

| | Comp. Ex. 1-1 | Comp. Ex. 1-2 | Ex. 1-1 | Ex. 1-2 | Ex. 1-3 | Ex. 1-4 | Ex. 1-5 | Ex. 1-6 |
|---|---|---|---|---|---|---|---|---|
| Water | 82.738 | 12.738 | 72.738 | 62.738 | 37.738 | 27.738 | 22.738 | 17.738 |
| Lecithin | | | | | | | | |
| Polyoxyethylene sorbitan monolaurate (20E.O) | 1 | 1 | 1 | 1 | 1 | 1 | 1 | 1 |
| Glyceryl stearate | | | | | | | | |
| C12-C15 alkyl benzoate | 5 | 5 | 5 | 5 | 5 | 5 | 5 | 5 |
| Coconut oil | | | | | | | | |
| Olive oil | | | | | | | | |
| Palm oil | | | | | | | | |
| Squalane | | | | | | | | |
| Camelia oil | | | | | | | | |
| Carbomer | 0.2 | 0.2 | 0.2 | 0.2 | 0.2 | 0.2 | 0.2 | 0.2 |
| Ethanol | 10 | 80 | 20 | 30 | 55 | 65 | 70 | 75 |
| Glycerin | 1 | 1 | 1 | 1 | 1 | 1 | 1 | 1 |
| Aminoethyl propanol | 0.062 | 0.062 | 0.062 | 0.062 | 0.062 | 0.062 | 0.062 | 0.062 |
| Formulation stability | Stable | Unstable | Stable | Stable | Stable | Stable | Stable | Stable |
| Mortality rate (%), determined 1 minute after inoculation with *Staphylococcus aureus* | -4 | 99.999 | 99.999 | 99.999 | 99.999 | 99.999 | 99.999 | 99.999 |

### Example 2. Evaluation of Formulation Stability Depending on Change in Ethanol Content and Type of Surfactant

Each disinfectant composition having an O/W emulsion formulation was prepared according to the composition (wt%) of the following Table 2 in the same manner as Example 1.

In addition, the formulation stability of each composition was evaluated in the same manner as Example 1.

As a result, when the ethanol content of a composition is high, the emulsion particles of the composition become unstable, and separation of the formulation occurs. However, it can be seen that when a sorbitan-based surfactant is used as a surfactant, as shown in Table 1, or a lecithin-based surfactant is used as a surfactant, as shown in Table 2, the composition shows a stable formulation.

Meanwhile, there was no significant change in the moisturizing feel of each composition depending on the type of a surfactant. The moisturizing feel was evaluated by allowing each of twenty male and female subjects suffering no skin diseases to load and rub 2g of each composition on the back of his/her hand, and to rub the back of his/her hand after drying the composition, and then by performing a survey about the moisturizing feel. The moisturizing feel was evaluated based on a score of 1 to 7 out of the list of socres shown below. The results of the moisturizing feel evaluation of each composition were compared using average values.
Score 1: Very dry after application.
Score 2: Dry after application.
Score 3: A bit dry after application.
Score 4: No feeling after application.
Score 5: A bit moist after application.
Score 6: Moist after application.
Score 7: Very moist after application.

**[Table 2]**

| | Comp. Ex. 2-1 | Comp. Ex. 2-2 | Comp. Ex. 2-3 | Comp. Ex. 2-4 | Comp. Ex. 2-5 | Comp. Ex. 2-6 | Ex. 2-1 |
|---|---|---|---|---|---|---|---|
| Water | 90.738 | 82.738 | 72.738 | 62.738 | 37.738 | 37.738 | 37.738 |
| Lecithin | | | | | | | 1 |
| PEG 100 stearate | | | | | | 1 | |
| Polyoxyethylene sorbitan monolaurate (20E.O) | | | | | | | |
| Glyceryl stearate | 1 | 1 | 1 | 1 | 1 | | |
| C12-C15 alkyl benzoate | 5 | 5 | 5 | 5 | 5 | 5 | 5 |
| Coconut oil | | | | | | | |
| Olive oil | | | | | | | |
| Palm oil | | | | | | | |
| Squalane | | | | | | | |
| Camelia oil | | | | | | | |
| Carbomer | 0.2 | 0.2 | 0.2 | 0.2 | 0.2 | 0.2 | 0.2 |
| Ethanol | 2 | 10 | 20 | 30 | 55 | 55 | 55 |
| Glycerin | 1 | 1 | 1 | 1 | 1 | 1 | 1 |
| Aminoethyl propanol | 0.062 | 0.062 | 0.062 | 0.062 | 0.062 | 0.062 | 0.062 |
| Formulation stability | Stable | Unstable | Unstable | Unstable | Unstable | Unstable | Stable |

### Example 3. Evaluation of Formulation Stability Depending on Change in Surfactant Content

Each disinfectant composition having an O/W emulsion formulation was prepared according to the composition (wt%) of the following Table 3 in the same manner as Example 1.

In addition, the formulation stability of each composition was evaluated in the same manner as Example 1.

As a result, it can be seen that when a composition has an excessively low content of surfactant, despite the use of a sorbitan-based surfactant, the emulsion particles become unstable. It can be also seen that when a composition has an excessively high content of surfactant, it undergoes solubilization, and thus cannot form an O/W formulation.

**[Table 3]**

| | Comp. Ex. 3-1 | Comp. Ex. 3-2 | Ex. 3-1 | Ex. 3-2 | Ex. 3-3 |
|---|---|---|---|---|---|
| Water | 38.638 | 33.738 | 38.438 | 36.738 | 35.738 |
| Lecithin | | | | | |
| Polyoxyethylene sorbitan monolaurate (20E.O) | 0.1 | 5 | 0.3 | 2 | 3 |
| Glyceryl stearate | | | | | |
| C12-C15 alkyl benzoate | 5 | 5 | 5 | 5 | 5 |
| Coconut oil | | | | | |
| Olive oil | | | | | |
| Palm oil | | | | | |
| Squalane | | | | | |
| Camelia oil | | | | | |
| Carbomer | 0.2 | 0.2 | 0.2 | 0.2 | 0.2 |
| Ethanol | 55 | 55 | 55 | 55 | 55 |
| Glycerin | 1 | 1 | 1 | 1 | 1 |
| Aminoethyl propanol | 0.062 | 0.062 | 0.062 | 0.062 | 0.062 |
| Formulation stability | Unstable | Unstable | Stable | Stable | Stable |

### Example 4. Evaluation of Formulation Stability and Moisturizing Feel Depending on Type of Oil

Each disinfectant composition having an O/W emulsion formulation was prepared according to the composition (wt%) of the following Table 4 and Table 5 in the same manner as Example 1.

In addition, the formulation stability of each composition was evaluated in the same manner as Example 1, and the moisturizing feel of each composition was evaluated in the same manner as Example 2. The result of the moisturizing feel evaluation of each composition is shown as average value.

As a result, it can be seen that use of a different type of oil shows a difference in moisturizing feel, and use of a C1-C15 alkyl benzoate, coconut oil, olive oil or palm oil provides a good or excellent moisturizing feel. Particularly, when a composition uses a C12-C15 alkyl benzoate, it shows the highest moisturizing feel.

**[Table 4]**

| | Comp. Ex. 4-1 | Comp. Ex. 4-2 | Ex. 4-1 | Ex. 4-2 | Ex. 4-3 | Ex. 4-4 |
|---|---|---|---|---|---|---|
| Water | 37.738 | 37.738 | 37.738 | 37.738 | 37.738 | 37.738 |
| Lecithin | | | | | | |
| Polyoxyethylene sorbitan monolaurate (20E.O) | 1 | 1 | 1 | 1 | 1 | 1 |
| Glyceryl stearate | | | | | | |
| C12-C15 alkyl benzoate | | | 5 | | | |
| Coconut oil | | | | 5 | | |
| Olive oil | | | | | 5 | |
| Palm oil | | | | | | 5 |
| Squalane | 5 | | | | | |
| Camelia oil | | 5 | | | | |
| Carbomer | 0.2 | 0.2 | 0.2 | 0.2 | 0.2 | 0.2 |
| Ethanol | 55 | 55 | 55 | 55 | 55 | 55 |
| Glycerin | 1 | 1 | 1 | 1 | 1 | 1 |
| Aminoethyl propanol | 0.062 | 0.062 | 0.062 | 0.062 | 0.062 | 0.062 |
| Formulation stability | Stable | Stable | Stable | Stable | Stable | Stable |
| Moisturizing feel (out of score 7) | 3 | 3 | 6 | 5 | 5 | 5 |

**[Table 5]**

| | Ex. 4-5 | Ex. 4-6 | Ex. 4-7 | Ex. 4-8 |
|---|---|---|---|---|
| Water | 37.738 | 37.738 | 37.738 | 37.738 |
| Lecithin | 1 | 1 | 1 | 1 |
| Polyoxyethylene sorbitan monolaurate (20E.O) | | | | |
| Glyceryl stearate | | | | |
| C12-C15 alkyl benzoate | 5 | | | |
| Coconut oil | | 5 | | |
| Olive oil | | | 5 | |
| Palm oil | | | | 5 |
| Squalane | | | | |
| Camelia oil | | | | |
| Carbomer | 0.2 | 0.2 | 0.2 | 0.2 |
| Ethanol | 55 | 55 | 55 | 55 |
| Glycerin | 1 | 1 | 1 | 1 |
| Aminoethyl propanol | 0.062 | 0.062 | 0.062 | 0.062 |
| Formulation stability | Stable | Stable | Stable | Stable |
| Moisturizing feel (out of score 7) | 7 | 6 | 5 | 5 |

### Example 5. Evaluation of Formulation Stability and Moisturizing Feel Depending on Change in Oil Content

Each disinfectant composition having an O/W emulsion formulation was prepared according to the composition (wt%) of the following Table 6 in the same manner as Example 1.

In addition, the formulation stability of each composition was evaluated in the same manner as Example 1, and the moisturizing feel of each composition was evaluated in the same manner as Example 2. The result of the moisturizing feel evaluation of each composition is shown as average value.

As a result, it can be seen that the compositions show a different level of formulation stability and moisturizing feel depending on a change in oil content. It is shown that increasing oil content does not increase the score of a moisturizing feel.

**[Table 6]**

| | Comp. Ex. 5-1 | Comp. Ex. 5-2 | Ex. 5-1 | Ex. 5-2 | Ex. 5-3 |
|---|---|---|---|---|---|
| Water | 41.738 | 26.738 | 40.738 | 36.738 | 27.738 |
| Lecithin | | | | | |
| Polyoxyethylene sorbitan monolaurate (20E.O) | 1 | 1 | 1 | 1 | 1 |
| Glyceryl stearate | | | | | |
| C12-C15 alkyl benzoate | 1 | 16 | 2 | 6 | 15 |
| Coconut oil | | | | | |
| Olive oil | | | | | |
| Palm oil | | | | | |
| Squalane | | | | | |
| Camelia oil | | | | | |
| Carbomer | 0.2 | 0.2 | 0.2 | 0.2 | 0.2 |
| Ethanol | 55 | 55 | 55 | 55 | 55 |
| Glycerin | 1 | 1 | 1 | 1 | 1 |
| Aminoethyl propanol | 0.062 | 0.062 | 0.062 | 0.062 | 0.062 |
| Formulation stability | Stable | Unstable | Stable | Stable | Stable |
| Moisturizing feel (out of score 7) | 3 | 4 | 6 | 6 | 5 |

The present disclosure has been described in detail. It will be apparent to those skilled in the art that the description proposed herein is just a preferable example for the purpose of illustrations only, not intended to limit the scope of the disclosure, so it should be understood that other equivalents and modifications could be made thereto without departing from the scope of the disclosure.

## Claims

1. An emulsion-type disinfectant composition comprising:
ethanol;
a surfactant;
oil; and
water,
wherein the content of ethanol is more than 10 wt% based on the total weight of the disinfectant composition.

2. The emulsion-type disinfectant composition according to claim 1, wherein the content of ethanol is 20 wt% or more, 30 wt% or more, 55 wt% or more, 60 wt% or more, 65 wt% or more, or 70 wt% or more, based on the total weight of the disinfectant composition.

3. The emulsion-type disinfectant composition according to claim 1, wherein the surfactant is at least one of a lecithin-based surfactant and a sorbitan-based surfactant.

4. The emulsion-type disinfectant composition according to claim 3, wherein the lecithin-based surfactant is at least one selected from the group consisting of lecithin, hydrogenated lecithin and synthetic lecithin.

5. The emulsion-type disinfectant composition according to claim 3, wherein the sorbitan-based surfactant is a sorbitan fatty acid ester.

6. The emulsion-type disinfectant composition according to claim 3, wherein the sorbitan-based surfactant is at least one selected from the group consisting of sorbitan olivate, sorbitan stearate, sorbitan isostearate, sorbitan tristearate, sorbitan sesquistearate, sorbitan palmitate, sorbitan laurate, sorbitan oleate, sorbitan sesquioleate, sorbitan trioleate, sorbitan stearate/sorbityl laurate, polyoxyethylene sorbitan monolaurate, Polysorbate 20, Polysorbate 40, Polysorbate 60, Polysorbate 61, Polysorbate 65, Polysorbate 80 and Polysorbate 85.

7. The emulsion-type disinfectant composition according to claim 1, wherein the surfactant is used in an amount of more than 0.1 wt% and less than 5 wt%, based on the total weight of the disinfectant composition.

8. The emulsion-type disinfectant composition according to claim 1, wherein the oil is at least one selected from the group consisting of C12-C15 alkyl benzoates, coconut oil, olive oil and palm oil.

9. The emulsion-type disinfectant composition according to claim 1, wherein the oil is used in an amount of more than 1 wt% and less than 16 wt%, based on the total weight of the disinfectant composition.

10. The emulsion-type disinfectant composition according to claim 1, wherein the surfactant and the oil are present in a ratio of 0.3 to 3 : 5 based on the weight ratio of the surfactant and oil components.

11. The emulsion-type disinfectant composition according to claim 1, which further comprises at least one additive selected from a thickener, polyol and a pH modifier.

12. The emulsion-type disinfectant composition according to claim 11, wherein the additive is used in an amount of 0.5-2.0 wt%, based on the total weight of the disinfectant composition.

13. The emulsion-type disinfectant composition according to claim 1, which has a viscosity of 100-10,000 cps.

14. The emulsion-type disinfectant composition according to claim 1, which has emulsion particles having an average size of 1-10 µm.

15. The emulsion-type disinfectant composition according to claim 1, which is an O/W emulsion formulation.

16. The emulsion-type disinfectant composition according to claim 1, which is a skin disinfectant composition.

17. The emulsion-type disinfectant composition according to claim 1, which is a hand disinfectant composition.

18. The emulsion-type disinfectant composition according to claim 1, which is a composition for external use on skin.
